# EUROPÄISCHE PATENTSCHRIFT

(11) **EP 3 448 160 B1**
(45) Veröffentlichungstag und Bekanntmachung des Hinweises auf die Patenterteilung: **27.08.2025**
(21) Anmeldenummer: 16721382.6
(22) Anmeldetag: 26.04.2016
(51) Int. Cl.: A22C 17/00, A22C 25/00

(54) **VERFAHREN UND VORRICHTUNG ZUR INSPEKTION DER BAUCHHÖHLE ENTWEIDETER FISCHE**
METHOD AND DEVICE FOR INSPECTING THE ABDOMINAL CAVITY OF GUTTED FISH
PROCÉDÉ ET DISPOSITIF D'INSPECTION DE LA CAVITÉ ABDOMINALE DE POISSONS ÉVIDÉS

(43) Veröffentlichungstag der Anmeldung: 06.03.2019
(73) Patentinhaber: Nordischer Maschinenbau Rud. Baader GmbH + Co. KG, 23560 Lübeck (DE); IHFood A/S, 2200 Copenhagen N (DK)
(72) Erfinder: STEFFENS, Alexander, 23566 Lübeck (DE); PAULSOHN, Carsten, 23568 Lübeck (DE); RASMUSSEN, Niels Tjørnly, 2650 Hvidovre (DK); JENSEN, Eigil Mølvig, 2300 Kopenhagen S (DK); NIELSEN, Ulrik, 1366 Kopenhagen K (DK)
(74) Vertreter: Stork Bamberger Patentanwälte PartmbB
(86) Internationale Anmeldenummer: PCT/EP2016/059307
(87) Internationale Veröffentlichungsnummer: WO 2017/186275

(56) Entgegenhaltungen:
- WO-A1-96/03887
- US-A1- 2004 023 612
- US-B1- 6 649 412
- KROMA A/S: "Gutmaster 3000 - The perfect gutting machine for medium sized fish", 18 May 2009 (2009-05-18), XP055335179, Retrieved from the Internet <URL:http://www.kroma.dk/wp-content/uploads/2015/06/090615_gm_3000.pdf> [retrieved on 20170113]
- KROMA A/S: "Visiomaster - The good result is now visible", 22 May 2008 (2008-05-22), Internet, XP055334652, Retrieved from the Internet <URL:http://www.odinmakine.com.tr/fileadmin/user_upload/BrosurMakinelerWebSitesi/8-SuurunleriislemeMakineleri/2-KromaAS/Katalog/12-VisionMaster.pdf> [retrieved on 20170112]
- ANONYMOUS: "Salmon Gutting Revolution - BAADER 144 / BAADER 1570 - Food Processing Technology", 9 March 2016 (2016-03-09), Internet, XP055334618, Retrieved from the Internet <URL:http://www.foodprocessing-technology.com/contractors/processing/baader/pressbaader-144-baader-1570.html> [retrieved on 20170112]

## Beschreibung

Die vorliegende Erfindung betrifft ein Verfahren zur optischen Inspektion entweideter Fische mit geöffneter Bauchhöhle. Des Weiteren betrifft die Erfindung ein Verfahren zum Entweiden von Fischen, die entlang einer Bearbeitungslinie in Längsrichtung mittels einer Fördereinrichtung gefördert werden.

Ferner betrifft die Erfindung eine Vorrichtung zur optischen Inspektion der geöffneten Bauchhöhle entweideter Fische sowie eine Vorrichtung zum Entweiden von Fischen, die entlang einer Bearbeitungslinie in Längsrichtung mittels einer Fördereinrichtung gefördert werden.

Derartige Vorrichtungen und Verfahren kommen bei der industriellen Fischverarbeitung zum Einsatz. In der Regel werden die Fische automatisch geschlachtet, indem die Bauchhöhle der Fische eröffnet und die Eingeweide aus dieser mittels entsprechender Werkzeuge automatisch entfernt werden. Das Entweideergebnis wird üblicherweise optisch überprüft. Hierzu wird die Bauchhöhle der Fische im Anschluss an den Entweideschritt mit einer Farbkamera inspiziert und das aufgenommene Bild der Bauchhöhle mittels bekannter Bildverarbeitungsalgorithmen auf mögliche verbleibende Blut- und/ oder Eingeweidereste untersucht. Derartigen Vorrichtungen und Verfahren sind zum Beispiel aus "Gutmaster 3000 - The perfect gutting machine for medium sized fish" und "Visiomaster - The good result is now visible" bekannt.

Diese bekannten Vorrichtungen und Verfahren weisen eine Reihe von Nachteilen auf. Aufgrund der Verwendung einer Farbkamera, die das einfallende Licht mittels Bayerfilter jeweils in die Grundfarben zerlegt, ist die effektive Auflösung gegenüber einer Monochrom-Kamera deutlich reduziert. Zum anderen sind entsprechend lange Belichtungszeiten erforderlich. Dies steht der Förderung einer möglichst hohen Fördergeschwindigkeit der Fische entgegen, da aufgrund der einzuhaltenden Belichtungszeit die maximale Fördergeschwindigkeit stark begrenzt ist. Eine Erhöhung der Fördergeschwindigkeit führt zu unscharfen Bildern der Bauchhöhle und so insgesamt zu einer geringen Verlässlichkeit der Bildauswertung. Ein weiterer Nachteil besteht darin, dass die Farbbildauswertung rechen- und damit zeitintensiv ist.

Es ist daher Aufgabe der vorliegenden Erfindung, eine Vorrichtung sowie ein Verfahren vorzuschlagen, die eine zuverlässige optische Inspektion der Bauchhöhle bei zugleich möglichst hohen Fördergeschwindigkeiten der Fische gewährleisten. Des Weiteren besteht die Aufgabe darin, eine Vorrichtung sowie ein Verfahren zum Entweiden von Fischen vorzuschlagen, die bei großer Fördergeschwindigkeit ein möglichst verlässliches Entweideergebnis ermöglichen.

Die Aufgabe wird durch die eingangs genannte Vorrichtung gelöst, wobei die Vorrichtung eine zum Fördern der Fische in Längsrichtung entlang einer Bearbeitungslinie eingerichtete Fördereinrichtung, zum Aufhalten der Bauchhöhle der Fische eingerichtete schwenkbare Bauchlappenhalteelemente, mindestens einen entlang der Bearbeitungslinie angeordneten optischen Bildgeber, der zum Aufnehmen mindestens einer mehrere Einzelbilder umfassenden Bildsequenz von Bauchhöhlenbereichen jeweils eines der Fische ausgebildet und eingerichtet ist, mindestens eine zum Beleuchten der Bauchhöhlenbereiche jeweils zu den Aufnahmezeitpunkten der Einzelbilder mit Licht jeweils vorbestimmter Spektralzusammensetzung eingerichtete Beleuchtungseinrichtung, eine zum Auswerten der Bildsequenz hinsichtlich vorgegebener Qualitätsparameter eingerichtete Auswerteeinheit, die ausgebildet ist, falls das Auswerten eine Abweichung von den vorgegebenen Qualitätsparametern ergibt, eine Ausschleuseeinrichtung anzusteuern, um den jeweiligen der Fische von der Bearbeitungslinie durch Ausschleusen zu entfernen, umfasst. Dies bietet den Vorteil, dass die Bauchhöhlenbereiche mittels der Beleuchtungseinrichtungen mit einer vorgegebenen Lichtfarbe bzw. einem Lichtwellenlängenbereich beleuchtet werden und die Einzelbilder von jeweils einem der optischen Bildgeber aufgenommen werden. Durch das Beleuchten mit farbigem Licht wird der jeweils interessierende Wellenlängenbereich mittels der optischen Bildgeber aufgenommen. Ein Farbfilter, beispielsweise ein Bayerfilter oder dergleichen, ist in dem optischen Bildgeber nicht mehr erforderlich. Dies kommt einerseits der Gesamtauflösung des jeweiligen der optischen Bildgeber zu Gute, andererseits wird die Intensität des einfallenden Lichts nicht durch etwaige Filter abgeschwächt. Dies begünstigt kurze Belichtungszeiten, so dass die Fische mit entsprechend hohen Fördergeschwindigkeiten transportiert werden können. Ein weiterer Vorteil besteht darin, dass aufgrund der aktiven Beleuchtung mittels der Beleuchtungseinrichtungen der Fisch bzw. dessen Bauchhöhle mit hochintensivem Licht ausgeleuchtet werden kann. Die Beleuchtungseinrichtungen sind vorzugsweise aus LED-Arrays gebildet. Die Beleuchtungseinrichtungen werden weiter bevorzugt jeweils nur für die Dauer der erforderlichen Belichtungszeit betrieben, so dass die auszuleuchtenden Bereiche blitzartig beleuchtet werden. Die vorliegende Erfindung eignet sich daher insbesondere zur optischen Inspektion von rasch bewegten Objekten.

Eine vorteilhafte Weiterbildung der Erfindung ist dadurch gekennzeichnet, dass die mindestens eine Beleuchtungseinrichtung eingerichtet ist, den Bauchhöhlenbereich zu den Aufnahmezeitpunkten jeweils mit Licht verschiedener Spektralbereiche zu beleuchten. Auf diese Weise ist es möglich, die Bauchhöhle der Fische nach beliebigen Qualitätsmerkmalen optimal angepasst zu inspizieren. Vorzugsweise sind die verwendeten Licht-Spektralbereiche auf die jeweiligen zu erkennenden Strukturen abgestimmt. Die eigentliche Auswertung der Bildsequenz erfolgt mittels üblicher Bildauswertealgorithmen, die entweder auf statistischen Methoden oder auf der Anwendung neuronaler Netze beruhen.

Gemäß einer bevorzugten Ausbildung umfasst die mindestens eine Beleuchtungseinrichtung eine Lichtquelle, die zur Aussendung von Licht eines schmalbandigen Spektrums ausgebildet ist. Auf diese Weise wird die Selektivität zur Erkennung bestimmter Strukturen weiter erhöht.

Erfindungsgemäß umfasst die Vorrichtung weitere Steuermittel, die ausgebildet und eingerichtet sind, den mindestens einen optischen Bildgeber derart zu steuern, dass der zeitliche Abstand der Aufnahmen zweier aufeinanderfolgender der Einzelbilder einer der Bildsequenzen derart gewählt ist, dass die beiden aufeinanderfolgenden Einzelbilder zumindest im Wesentlichen denselben Bauchhöhlenbereich abbilden. Dies wirkt sich besonders vorteilhaft auf die Komplexität der Bildauswertung aus. Aufgrund des nun möglichen geringen zeitlichen Abstands zwischen den Aufnahmen führt die kontinuierliche Weiterbewegung des jeweiligen Fisches nicht zu einer örtlichen Verschiebung der Einzelbilder. Ein andernfalls erforderliches Korrigieren - englisch "mapping" - der Bildlage, um die Einzelbilder zur Deckung zu bringen, kann daher entfallen. Dies wirkt sich positiv auf die zur Auswertung der Bildsequenz benötigten Rechenzeit aus.

Erfindungsgemäß sind entlang der Bearbeitungslinie mehrere der optischen Bildgeber jeweils separat angeordnet und eingerichtet, jedes der Einzelbilder mit einem der optischen Bildgeber aufzunehmen. Auf diese Weise ist eine zuverlässige Inspektion auch bei hohen Fördergeschwindigkeiten stets gewährleistet.

Gemäß einer vorteilhaften Ausgestaltung der Erfindung ist jedem der optischen Bildgeber eine separate Beleuchtungseinrichtung zugeordnet, das eingerichtet ist, die Bauchhöhlenbereiche zu den jeweiligen Aufnahmezeitpunkten zu beleuchten. Wie bereits erwähnt, können so die entsprechenden Bereiche und Strukturen mit hoher Erkennungsgenauigkeit untersucht werden.

Eine weitere vorteilhafte Ausgestaltung zeichnet sich dadurch aus, dass der mindestens eine optische Bildgeber als Schwarz-weiß-Kamera ausgebildet ist. Der Einsatz von Schwarz-weiß-Kameras ermöglicht hochaufgelöste Bilder und somit eine entsprechend hohe Genauigkeit der Bauchhöhleninspektion. Zudem sind diese entsprechend lichtempfindlich, so dass die genannten kurzen Belichtungszeiten bzw. hohen Fördergeschwindigkeiten realisierbar sind.

Gemäß einer weiteren bevorzugten Ausbildung ist der mindestens eine optische Bildgeber derart angeordnet, dass mindestens eines der Einzelbilder mit senkrechter oder zumindest im Wesentlichen senkrechter Blickrichtung auf die Bauchhöhle aufgenommen wird. Auf diese Weise kann der gesamte Bereich der Bauchhöhle optimal ausgeleuchtet und mögliche verbliebene Reste zuverlässig erkannt werden.

Eine weitere Ausbildung der Erfindung sieht vor, dass mindestens ein weiterer optischer Bildgeber derart angeordnet ist, dass mindestens ein weiteres der Einzelbilder mit gegenüber der senkrechten Blickrichtung geneigter Blickrichtung aufgenommen wird. Dies ermöglicht einerseits die Erkennung von bei senkrechter Beleuchtung verschatteten Bereichen, so dass insgesamt die Erkennungsqualität und damit die Zuverlässigkeit der Bauchhöhleninspektion erhöht wird.

Vorteilhafterweise ist der mindestens eine weitere optische Bildgeber derart geneigt angeordnet, dass die Aufnahme in der geneigten Blickrichtung in Kopfrichtung der Fische erfolgt. Mit anderen Worten wird ein Schrägbild mit Blickrichtung vom Schwanz zum Kopf hin aufgenommen. Dies bietet neben dem zuvor genannten Vorteil der Inspektion verschatteter Bereiche den Zusatznutzen, auch den kopfseitigen Bereich der Bauchhöhle explizit inspizieren zu können.

Gemäß einer bevorzugten Ausbildung der Erfindung ist die Auswerteeinheit eingerichtet, die Einzelbilder der Bildsequenz zu einem Gesamtbild zu überlagern. Das Gesamtbild dient beispielsweise als Kontrollbild, um den Inspektionsvorgang stichprobenartig zu überprüfen. Alternativ wird das Gesamtbild als Ausgangspunkt für die optische Inspektion herangezogen.

Die Aufgabe wird auch durch die eingangs genannte Vorrichtung zum Entweiden von Fischen gelöst, wobei die Fische entlang einer Bearbeitungslinie in Längsrichtung mittels einer Fördereinrichtung gefördert werden, umfassend zum Öffnen der Bauchhöhle der Fische eingerichtete Öffnungswerkzeuge, zum Ausräumen der Bauchhöhle ausgebildete Entweidungswerkzeuge und eine Vorrichtung zur anschließenden optischen Inspektion der Bauchhöhle der entweideten Fische wie zuvor beschrieben. Die Kombination aus dem erfindungsgemäßen Verfahren zur optischen Inspektion der Bauchhöhle und dem vorgeschalteten Entweidevorgang gewährleistet einen Produktionsablauf hoher Güte, bei dem sichergestellt ist, dass die Fische nach dem Entweidevorgang den gestellten Qualitätsanforderungen hinsichtlich einer gereinigten Bauchhöhle entsprechen.

Des Weiteren wird die Aufgabe durch das eingangs genannte Verfahren gelöst, umfassend Fördern der Fische in Längsrichtung entlang einer Bearbeitungslinie mittels einer Fördereinrichtung, Einschwenken zum Aufhalten der Bauchhöhle der Fische eingerichteter Bauchlappenhalteelemente, Aufnehmen mindestens einer mehrere Einzelbilder umfassenden Bildsequenz von Bauchhöhlenbereichen jeweils eines der Fische mittels mindestens einem entlang der Bearbeitungslinie angeordnetem optischen Bildgeber, Beleuchten der Bauchhöhlenbereiche jeweils zu den Aufnahmezeitpunkten der Einzelbilder mittels mindestens einer Beleuchtungseinrichtung mit Licht jeweils vorbestimmter Spektralzusammensetzung, Auswerten der Bildsequenz hinsichtlich vorgegebener Qualitätsparameter und falls das Auswerten eine Abweichung von den vorgegebenen Qualitätsparametern ergibt, Entfernen des jeweiligen der Fische von der Bearbeitungslinie durch Ausschleusen mittels einer Ausschleuseeinrichtung.

Die mit dem erfindungsgemäßen Verfahren verbundenen Vorteile sind bereits zuvor im Zusammenhang mit der erfindungsgemäßen Vorrichtung detailliert erläutert worden. Zur Vermeidung von Wiederholungen wird auf die entsprechenden Textpassagen verwiesen. Dies gilt ebenso für die im Folgenden genannten bevorzugten Ausführungen des erfindungsgemäßen Verfahrens.

Eine bevorzugte Ausbildung der Erfindung zeichnet sich dadurch aus, dass das Beleuchten zu den Aufnahmezeitpunkten jeweils mit Licht verschiedener Spektralbereiche erfolgt. Vorzugsweise erfolgt das Beleuchten mit Licht eines schmalbandigen Spektrums.

Erfindungsgemäß ist der zeitliche Abstand der Aufnahme zweier aufeinanderfolgender der Einzelbilder einer der Bildsequenzen derart gewählt, dass die beiden aufeinanderfolgenden Einzelbilder zumindest im Wesentlichen denselben Bauchhöhlenbereich abbilden.

Erfindungsgemäß ist vorgesehen, dass jedes der Einzelbilder mit einem separaten optischen Bildgeber aufgenommen wird.

Vorteilhafter Weise ist jedem der optischen Bildgeber eine separate Beleuchtungseinrichtung zugeordnet, mittels dessen die Beleuchtung zu den jeweiligen Aufnahmezeitpunkten erfolgt.

Eine bevorzugte Ausbildung der Erfindung zeichnet sich dadurch aus, dass die Aufnahme der Einzelbilder in schwarz-weiß erfolgt.

Gemäß einer vorteilhaften Ausgestaltung der Erfindung wird mindestens eines der Einzelbilder mit einem der optischen Bildgeber mit senkrechter oder zumindest im Wesentlichen senkrechter Blickrichtung auf die Bauchhöhle aufgenommen.

Weiter bevorzugt ist vorgesehen, dass mindestens ein weiteres der Einzelbilder mit einem weiteren der optischen Bildgeber mit gegenüber der senkrechten Blickrichtung geneigter Blickrichtung aufgenommen wird. Insbesondere ist es vorteilhaft, dass die Aufnahme in der geneigten Blickrichtung in Kopfrichtung der Fische erfolgt.

Eine bevorzugte Ausbildung der Erfindung sieht vor, Einzelbilder der Bildsequenz zu einem Gesamtbild zu überlagern.

Die Aufgabe wird ferner durch das eingangs genannte Verfahren zum Entweiden von Fischen, die entlang einer Bearbeitungslinie in Längsrichtung mittels einer Fördereinrichtung gefördert werden, gelöst, wobei das Verfahren das Öffnen der Bauchhöhle der Fische, Ausräumen der Bauchhöhle mittels Entweidewerkzeugen und anschließende optische Inspektion der Bauchhöhle der entweideten Fische mittels des zuvor beschriebenen Verfahrens umfasst.

Weitere bevorzugte und/oder zweckmäßige Merkmale und Ausgestaltungen der Erfindung ergeben sich aus den Unteransprüchen und der Beschreibung. Besonders bevorzugte Ausführungsformen werden anhand der beigefügten Zeichnung näher erläutert. In der Zeichnung zeigt:
- Fig. 1: eine schematische Darstellung der erfindungsgemäßen Vorrichtung in Seitenansicht,
- Fig. 2: eine schematische Darstellung eines ersten erfindungsgemäßen Verfahrensschritts im Detail,
- Fig. 3: eine schematische Darstellung eines zweiten erfindungsgemäßen Verfahrensschritts im Detail,
- Fig. 4: eine schematische Detailansicht mit einem Bauchlappenhalteelement in einer Warteposition der erfindungsgemäßen Vorrichtung in Seitenansicht,
- Fig. 5: eine schematische Detailansicht mit einem Bauchlappenhalteelement in einer Arbeitsposition der erfindungsgemäßen Vorrichtung in Seitenansicht,
- Fig. 6: eine perspektivische Ansicht der in Fig. 4 dargestellten Detailansicht und
- Fig. 7: eine perspektivische Ansicht der in Fig.5 dargestellten Detailansicht.

Im Folgenden werden die erfindungsgemäße Vorrichtung sowie das erfindungsgemäße Verfahren anhand der Zeichnung näher beschrieben. Zur Vermeidung von Wiederholungen wird auch das erfindungsgemäße Verfahren anhand der erfindungsgemäßen Vorrichtung erläutert. Die zur Vorrichtung gemachten Ausführungen gelten daher in analoger Weise auch für das erfindungsgemäße Verfahren.

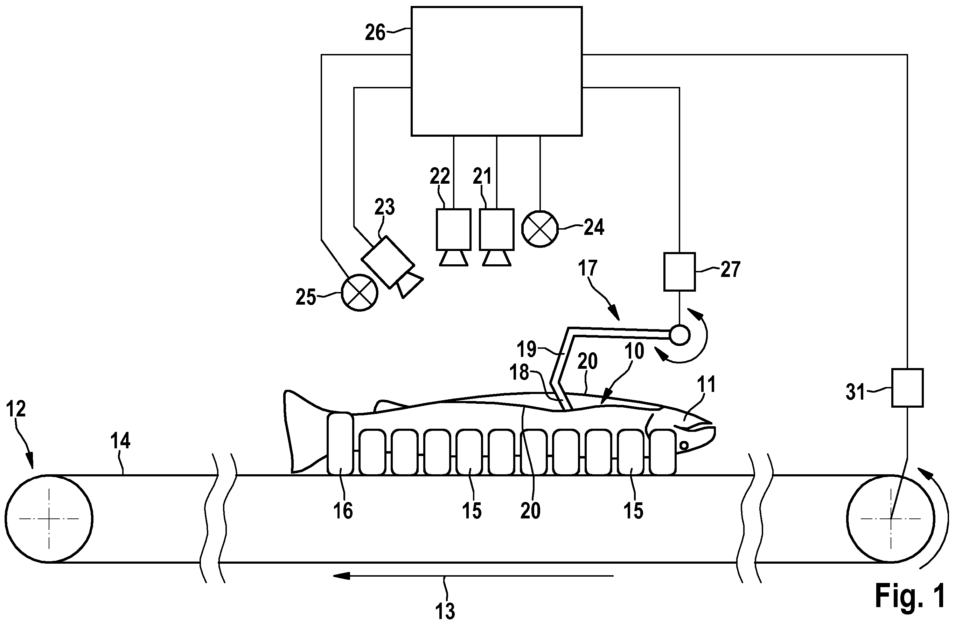

In der Figur 1 ist eine schematische Darstellung der erfindungsgemäßen Vorrichtung in Seitenansicht gezeigt. Die Vorrichtung ist zur optischen Inspektion der geöffneten Bauchhöhle 10 entweideter Fische 11 ausgebildet und eingerichtet. Die Vorrichtung umfasst eine zum Fördern der Fische 11 in Längsrichtung 13 entlang einer Bearbeitungslinie eingerichtete Fördereinrichtung 12. Die Fördereinrichtung 12 ist beispielsweise als umlaufendes Förderband 14 mit Halte- und Zentriermittel 15 ausgebildet. Die Halte- und Zentriermittel 15 sind eingerichtet, die Fische 11 in Rückenlage zu halten und auszurichten, so dass diese mit der geöffneten Bauchhöhle 10 nach oben zeigend in der Längsrichtung 13 gefördert werden. Vorzugsweise sind die Halte- und Zentriermittel 15 V-förmig ausgebildet. Jeweils eines der Halte- und Zentriermittel ist als Schwanzklammer 16 ausgebildet, die den jeweiligen der Fische 11 gegen ein Verrutschen in der Längsrichtung 13 sichert.

Die erfindungsgemäße Vorrichtung umfasst weiter zum Aufhalten der Bauchhöhle 10 der Fische eingerichtete schwenkbare Bauchlappenhalteelemente 17. Die Bauchlappenhalteelemente 17 umfassen vorzugsweise einen im Wesentlichen V-förmigen Spreizbügel 18, der über einen Schwenkhebel 19 in die Bauchhöhle 10 hinein und aus dieser wieder heraus bewegt wird. Die Bauchlappenhalteelemente 17 sind eingerichtet und ausgebildet, die Bauchlappen der geöffneten Bauchhöhle 10 der Fische 11 auseinanderzuspreizen.

An der Bearbeitungslinie ist mindestens ein optischer Bildgeber angeordnet, beispielsweise - wie in Figur 1 gezeigt - ein erster Bildgeber 21, ein zweiter Bildgeber 22 und ein dritter Bildgeber 23. Die vorliegende Erfindung ist jedoch nicht auf die gezeigte Anzahl von Bildgebern 21, 22, 23 beschränkt, sondern kann alternativ auch weniger oder mehr der Bildgeber 21, 22, 23 umfassen. Die Bildgeber 21, 22, 23 sind zum Aufnehmen mindestens einer mehrere Einzelbilder umfassenden Bildsequenz von Bauchhöhlenbereichen jeweils eines der Fische 11 ausgebildet und eingerichtet.

Mittels einer ersten Beleuchtungseinrichtung 24 sowie einer zweiten Beleuchtungseinrichtung 25 werden die Bauchhöhlenbereiche jeweils zu den Aufnahmezeitpunkten der Einzelbilder mit Licht jeweils vorbestimmter Spektralzusammensetzung beleuchtet. Die Anzahl der Beleuchtungsvorrichtung ist nicht - wie in Figur 1 gezeigt - auf nur zwei beschränkt. Alternativ ist es auch möglich nur eine einzige der Beleuchtungseinrichtungen 24, 25 einzusetzen oder mehr als zwei der Beleuchtungseinrichtungen 24, 25 zu verwenden.

Eine Auswerteeinheit 26 ist eingerichtet und ausgebildet, die Bildsequenz hinsichtlich vorgegebener Qualitätsparameter auszuwerten, und falls das Auswerten eine Abwiechung von den vorgegebenen Qualitätsparametern ergibt, eine - in der Zeichnung nicht gezeigte - Ausschleuseeinrichtung anzusteuern, um den jeweiligen der Fische 11 von der Bearbeitungslinie durch Ausschleusen zu entfernen. Die vorgegebenen Qualitätsparameter beziehen sich beispielsweise auf optische Vergleichswerte, die Aufschluss darüber geben, inwieweit bzw. zu welchem Grad die Innereien bereits entfernt worden sind. Dies umfasst auch Vergleichswerte, die Blutreste oder Bindegewebsreste betreffen. Zudem umfassen die Qualitätsparameter Größen, um die korrekte Lage des Bauchschnitts zu überprüfen.

Die Auswerteeinheit 26 ist mit den optischen Bildgebern 21, 22, 23, der ersten und zweiten Beleuchtungseinrichtungen 24, 25, einem Aktor 27 zum Bewegen der Bauchlappenhalteelemente 17 sowie vorzugsweise mit einem Antrieb 31 zum Antreiben der Fördereinrichtung 12 verbunden, so dass die Auswerteeinheit 26 die vorgenannten Komponenten steuern bzw. regeln kann. Die Verbindungen sind vorzugsweise als elektrische Verbindungen ausgebildet.

Die erste Beleuchtungseinrichtung 24 und die zweite Beleuchtungseinrichtung 25 sind jeweils eingerichtet, den Bauchhöhlenbereich zu den Aufnahmezeitpunkten jeweils mit Licht verschiedener Spektralbereiche zu beleuchten. Die Spektralbereiche umfassen jeweils einen Ausschnitt des sichtbaren bzw. infraroten Wellenlängenspektrums.

Bevorzugt umfassen die erste Beleuchtungseinrichtung 24 und die zweite Beleuchtungseinrichtung 25 jeweils eine Lichtquelle, die zur Aussendung von Licht eines schmalbandigen Spektrums ausgebildet ist. Schmalbandig bedeutet, dass das Licht einen Spektralbereich mit einer Wellenlänge kleiner 100 nm abdeckt. Besonders bevorzugt ist das Licht jeweils monochromatisch.

Die Auswerteeinheit 26 umfasst weiter Steuermittel, die ausgebildet und eingerichtet sind, die Bildgeber 21, 22, 23 derart zu steuern, dass der zeitliche Abstand der Aufnahmen zweier aufeinanderfolgender der Einzelbilder einer der Bildsequenzen derart gewählt ist, dass die beiden aufeinanderfolgenden Einzelbilder zumindest im Wesentlichen denselben Bauchhöhlenbereich abbilden. Mit anderen Worten sind die Steuermittel eingerichtet, in Abstimmung mit der eingestellten Fördergeschwindigkeit der Fördereinrichtung 12 die Aufnahme der Einzelbilder in einem solchen zeitlichen Abstand aufzunehmen, dass die jeweiligen der Einzelbilder im Wesentlichen denselben Bereich der Bauchhöhle 10 zeigen.

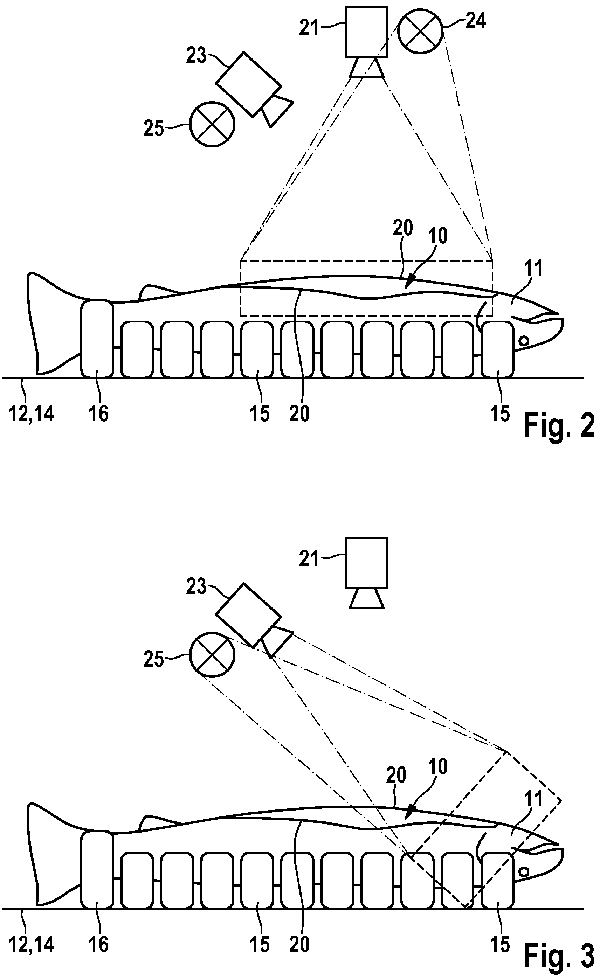

Wie aus den Figuren 2 und 3 ersichtlich, sind entlang der Bearbeitungslinie mehrere der optischen Bildgeber 21, 22, 23 angeordnet, wobei in den Detailansichten der Figuren 2 und 3 jeweils nur der erste Bildgeber 21 sowie der dritte Bildgeber 23 dargestellt ist. Die optischen Bildgeber 21, 22, 23 sind vorzugsweise jeweils separat angeordnet und eingerichtet, um jedes der Einzelbilder mit einem der optischen Bildgeber 21, 22, 23 aufzunehmen. Aus Gründen der besseren Übersicht, sind die Bauchlappenhalteelemente 17, die die Bauchhöhle 10 des jeweiligen Fisches 11 zur optischen Inspektion offenhalten, in den Figuren 2 und 3 nicht gezeigt.

Besonders bevorzugt ist jedem der optischen Bildgeber 21, 22, 23 eine separate Beleuchtungseinrichtung zugeordnet, die eingerichtet sind, die Bauchhöhlenbereiche zu den jeweiligen Aufnahmezeitpunkten zu beleuchten. Wie in den Figuren 2 und 3 dargestellt, ist dem ersten Bildgeber 21 die erste Beleuchtungseinrichtung 24 sowie dem dritten Bildgeber 23 die zweite Beleuchtungseinrichtung 25 zugeordnet.

Gemäß einer vorteilhaften Ausgestaltung der Erfindung sind die Bildgeber 21, 22, 23 jeweils als Schwarz-weiß-Kamera bzw. als Monochromkamera ausgebildet. Alternativ ist jeweils einer oder mehrere der Bildgeber 21, 22, 23 als Farbkamera ausgebildet.

Vorzugsweise ist mindestens einer der optischen Bildgeber 21, 22, 23 derart angeordnet, dass mindestens eines der Einzelbilder mit senkrechter oder zumindest im Wesentlichen senkrechter Blickrichtung auf die Bauchhöhle 10 aufgenommen wird. Hierzu ist beispielsweise der erste Bildgeber 21 sowie die erste Beleuchtungseinrichtung 24 - wie in Figur 2 gezeigt - oberhalb der Fördereinrichtung 12 angeordnet.

Wie in Figur 3 gezeigt, ist mindestens ein weiterer optischer Bildgeber, beispielsweise der dritte Bildgeber 23 derart angeordnet, dass mindestens ein weiteres der Einzelbilder mit gegenüber der senkrechten Blickrichtung geneigter Blickrichtung aufgenommen wird. Insbesondere ist der dritte Bildgeber 23 derart geneigt angeordnet, dass die Aufnahme in der geneigten Blickrichtung in Kopfrichtung der Fische erfolgt.

Die Auswerteeinheit 26 ist insbesondere eingerichtet, die Einzelbilder der Bildsequenz zu einem Gesamtbild zu überlagern. Das Gesamtbild kann optional zu Kontrollzwecken angezeigt werden. Weiter wird das Gesamtbild zur Qualitätskontrolle herangezogen. Die Auswerteeinheit 26 ist bevorzugt dazu eingerichtet, die Bildsequenz durch unterschiedliche Gewichtung der Einzelbilder zu einem Falschfarbenbild zusammenzusetzen.

Insbesondere die Bildgeber 21, 22, 23 sowie die Beleuchtungseinrichtungen 24, 25 sind vorzugsweise in einem Gehäuse 28 mit mindestens einer Sichtscheibe 30 gekapselt angeordnet.

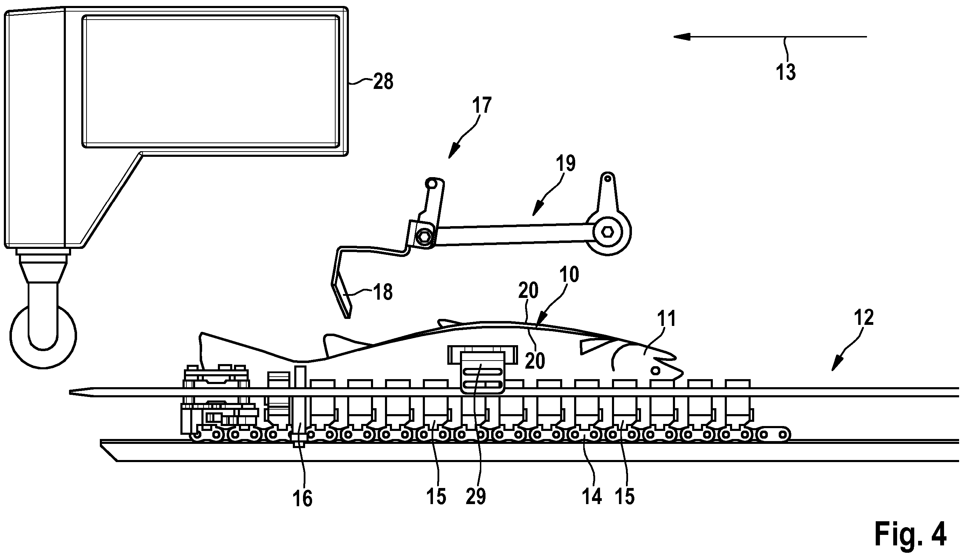

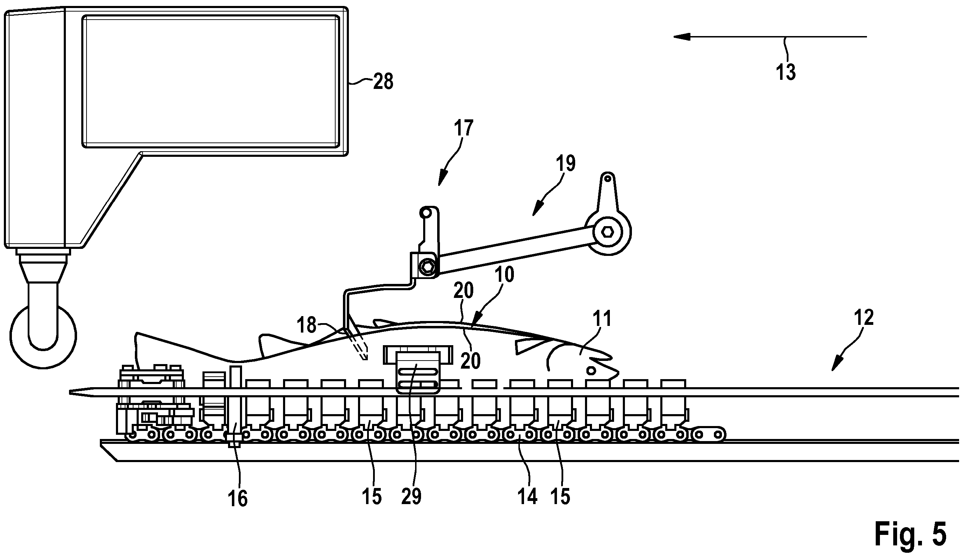

In der Figur 5 ist eine schematische Detailansicht mit den Bauchlappenhalteelementen 17 in einer Arbeitsposition der erfindungsgemäßen Vorrichtung in Seitenansicht gezeigt. Die Bauchlappenhalteelemente 17 sind in die Bauchhöhle eingesteuert und halten die Bauchlappen 20 seitlich offen, so dass die Bauchhöhle inspiziert werden kann. In Figur 4 ist gezeigt, wie sich die Bauchlappenhalteelemente 17 in einer Warteposition außerhalb der Bauchhöhle befinden.

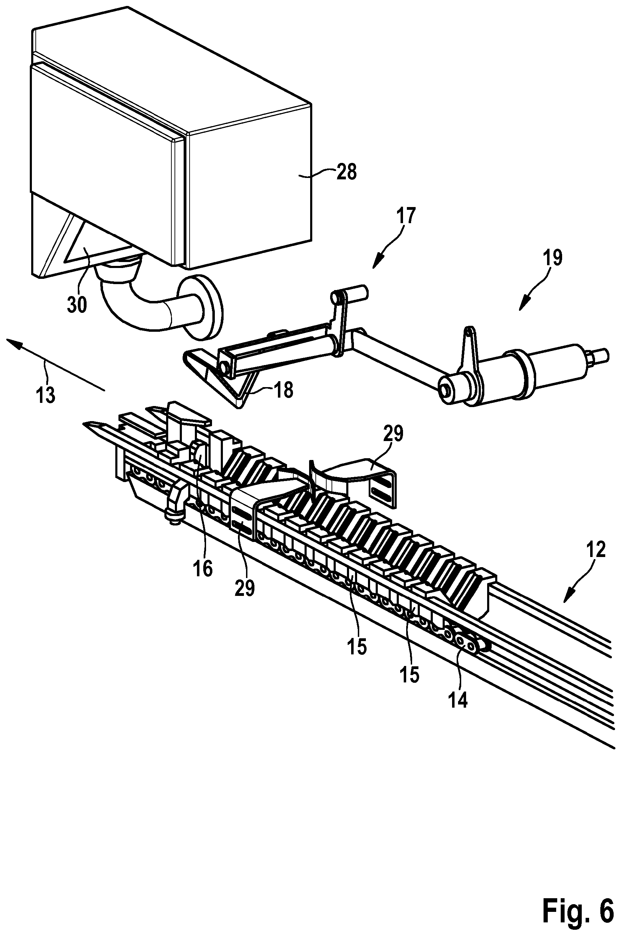

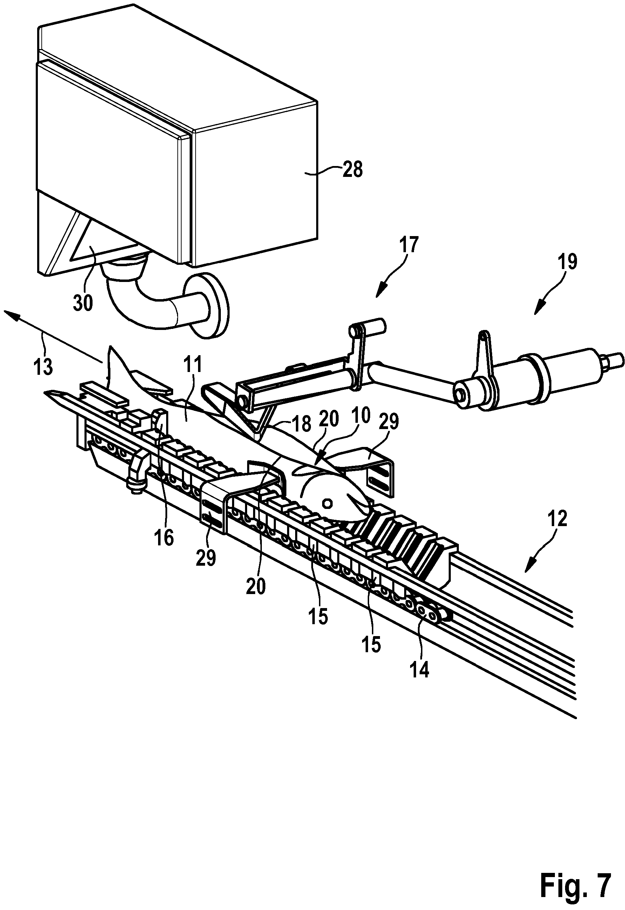

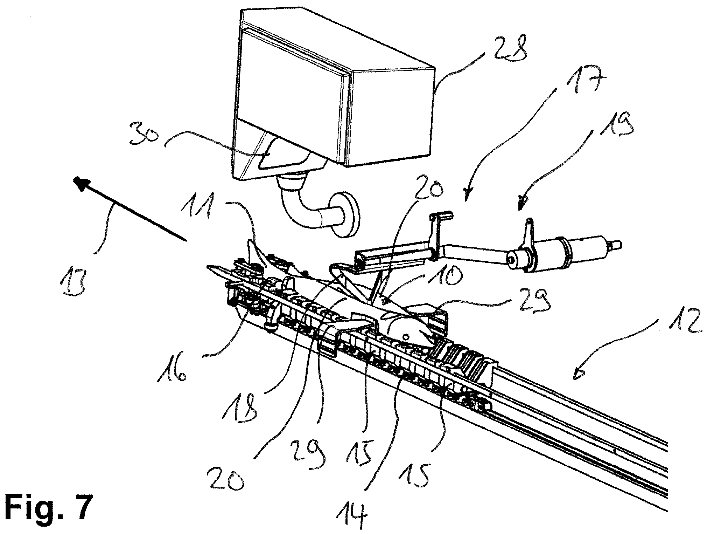

Die Figuren 6 und 7 zeigen jeweils eine perspektivische Ansicht der Detailansichten der Figuren 4 und 5. Die Figuren 4 bis 7 zeigen jeweils eine Zentrierhilfe 29, mittels derer die Fische 11 jeweils seitlich geführt und somit mittig zentriert werden.

Die vorliegende Erfindung umfasst weiter eine Vorrichtung zum Entweiden von Fischen 11, die entlang einer Bearbeitungslinie in Längsrichtung 13 mittels der Fördereinrichtung 12 gefördert werden. Die Vorrichtung umfasst zum Öffnen der Bauchhöhle 10 der Fische 11 eingerichtete Öffnungswerkzeuge, zum Ausräumen der Bauchhöhle 10 ausgebildete Entweidungswerkzeuge und eine Vorrichtung zur anschließenden optischen Inspektion der Bauchhöhle 10 der entweideten Fische 11 wie zuvor beschrieben. Derartige Vorrichtungen zum Öffnen der Bauchhöhle sowie die Entweidewerkzeuge sind beispielsweise aus den Dokumenten EP 1003378 A1 und EP 1411775 bekannt und werden daher nicht weiter im Detail beschrieben.

## Patentansprüche

1. Verfahren zur optischen Inspektion entweideter Fische (11) mit geöffneter Bauchhöhle (10), umfassend
Fördern der Fische (11) in Längsrichtung (13) entlang einer Bearbeitungslinie mittels einer Fördereinrichtung (12),
Einschwenken zum Aufhalten der Bauchhöhle (10) der Fische (11) eingerichtete Bauchlappenhalteelemente (17),
Aufnehmen mindestens einer mehrere Einzelbilder umfassenden Bildsequenz von Bauchhöhlenbereichen jeweils eines der Fische (11) mittels mindestens einem entlang der Bearbeitungslinie angeordnetem optischen Bildgeber (21, 22, 23),
Beleuchten der Bauchhöhlenbereiche jeweils zu den Aufnahmezeitpunkten der Einzelbilder mittels mindestens einer Beleuchtungseinrichtung (24, 25) mit Licht jeweils vorbestimmter Spektralzusammensetzung,
Auswerten der Bildsequenz hinsichtlich vorgegebener Qualitätsparameter und falls das Auswerten eine Abweichung von den vorgegebenen Qualitätsparametern ergibt, Entfernen des jeweiligen der Fische (11) von der Bearbeitungslinie durch Ausschleusen mittels einer Ausschleuseeinrichtung, wobei
jedes der Einzelbilder mit einem separaten optischen Bildgeber (21, 22, 23) aufgenommen wird wobei
der zeitliche Abstand der Aufnahme zweier aufeinanderfolgender der Einzelbilder einer der Bildsequenzen derart gewählt ist, dass die beiden aufeinanderfolgenden Einzelbilder zumindest im Wesentlichen denselben Bauchhöhlenbereich abbilden.

2. Verfahren nach Anspruch 1, **dadurch gekennzeichnet, dass** das Beleuchten zu den Aufnahmezeitpunkten jeweils mit Licht verschiedener Spektralbereiche erfolgt.

3. Verfahren nach Anspruch 2, **dadurch gekennzeichnet, dass** das Beleuchten mit Licht eines schmalbandigen Spektrums erfolgt.

4. Verfahren nach Anspruch 1 bis 3, **dadurch gekennzeichnet, dass** jedem der optischen Bildgeber (21, 22, 23) eine separate Beleuchtungseinrichtung (24, 25) zugeordnet ist, mittels dessen die Beleuchtung zu den jeweiligen Aufnahmezeitpunkten erfolgt.

5. Verfahren nach einem der Ansprüche 1 bis 4, **dadurch gekennzeichnet, dass** die Aufnahme der Einzelbilder in schwarz-weiß erfolgt.

6. Verfahren nach einem der Ansprüche 1 bis 5, **dadurch gekennzeichnet, dass** mindestens eines der Einzelbilder mit einem der optischen Bildgeber (21, 22, 23) mit senkrechter oder zumindest im Wesentlichen senkrechter Blickrichtung auf die Bauchhöhle (10) aufgenommen wird.

7. Verfahren nach Anspruch 6, **dadurch gekennzeichnet, dass** mindestens ein weiteres der Einzelbilder mit einem weiteren der optischen Bildgeber (23) mit gegenüber der senkrechten Blickrichtung geneigter Blickrichtung aufgenommen wird.

8. Verfahren nach Anspruch 7, **dadurch gekennzeichnet, dass** die Aufnahme in der geneigten Blickrichtung in Kopfrichtung der Fische (11) erfolgt.

9. Verfahren nach einem der Ansprüche 2 bis 8, **gekennzeichnet durch** Überlagern der Einzelbilder der Bildsequenz zu einem Gesamtbild.

10. Verfahren zum Entweiden von Fischen (11), die entlang einer Bearbeitungslinie in Längsrichtung (13) mittels einer Fördereinrichtung (12) gefördert werden, Öffnen der Bauchhöhle (10) der Fische (11), Ausräumen der Bauchhöhle (10) mittels Entweidewerkzeugen und anschließende optische Inspektion der Bauchhöhle (10) der entweideten Fische (11) mittels eines Verfahrens nach einem der Ansprüche 1 bis 9.

11. Vorrichtung zur optischen Inspektion der geöffneten Bauchhöhle (10) entweideter Fische (11), umfassend
eine zum Fördern der Fische (11) in Längsrichtung entlang einer Bearbeitungslinie eingerichtete Fördereinrichtung (12),
zum Aufhalten der Bauchhöhle (10) der Fische eingerichtete schwenkbare Bauchlappenhalteelemente (17),
mindestens einen entlang der Bearbeitungslinie angeordneten optischen Bildgeber (21, 22, 23), der zum Aufnehmen mindestens einer mehrere Einzelbilder umfassenden Bildsequenz von Bauchhöhlenbereichen jeweils eines der Fische (11) ausgebildet und eingerichtet ist,
mindestens eine zum Beleuchten der Bauchhöhlenbereiche jeweils zu den Aufnahmezeitpunkten der Einzelbilder mit Licht jeweils vorbestimmter Spektralzusammensetzung eingerichtete Beleuchtungseinrichtung (24, 25),
eine zum Auswerten der Bildsequenz hinsichtlich vorgegebener Qualitätsparameter eingerichtete Auswerteeinheit (26), die ausgebildet ist, falls das Auswerten eine Abweichung von den vorgegebenen Qualitätsparametern ergibt, eine Ausschleuseeinrichtung anzusteuern, um den jeweiligen der Fische (11) von der Bearbeitungslinie durch Ausschleusen zu entfernen, wobei
entlang der Bearbeitungslinie mehrere der optischen Bildgeber (21, 22, 23) jeweils separat angeordnet und eingerichtet sind, um jedes der Einzelbilder mit einem der optischen Bildgeber (21, 22, 23) aufzunehmen, sowie
Steuermittel, die ausgebildet und eingerichtet sind, den mindestens einen optischen Bildgeber (21, 22, 23) derart zu steuern, dass der zeitliche Abstand der Aufnahmen zweier aufeinanderfolgender der Einzelbilder einer der Bildsequenzen derart gewählt ist, dass die beiden aufeinanderfolgenden Einzelbilder zumindest im Wesentlichen denselben Bauchhöhlenbereich abbilden.

12. Vorrichtung nach Anspruch 11, **dadurch gekennzeichnet, dass** die mindestens eine Beleuchtungseinrichtung (24, 25) eingerichtet ist, den Bauchhöhlenbereich zu den Aufnahmezeitpunkten jeweils mit Licht verschiedener Spektralbereiche zu beleuchten.

13. Vorrichtung nach Anspruch 12, **dadurch gekennzeichnet, dass** die mindestens eine Beleuchtungseinrichtung (24, 25) eine Lichtquelle umfasst, die zur Aussendung von Licht eines schmalbandigen Spektrums ausgebildet ist.

14. Vorrichtung nach Anspruch 11, **dadurch gekennzeichnet, dass** jedem der optischen Bildgeber (21, 22, 23) ein separates Beleuchtungsmittel (24, 25) zugeordnet ist, die eingerichtet sind, die Bauchhöhlenbereiche zu den jeweiligen Aufnahmezeitpunkten zu beleuchten.

15. Vorrichtung nach einem der Ansprüche 11 bis 14, **dadurch gekennzeichnet, dass** der mindestens eine optische Bildgeber (21, 22, 23) als Schwarz-weiß-Kamera ausgebildet ist.

16. Vorrichtung nach einem der Ansprüche 11 bis 15, **dadurch gekennzeichnet, dass** der mindestens eine optische Bildgeber (21, 22, 23) derart angeordnet ist, dass mindestens eines der Einzelbilder mit senkrechter oder zumindest im Wesentlichen senkrechter Blickrichtung auf die Bauchhöhle (10) aufgenommen wird.

17. Vorrichtung nach Anspruch 16, **dadurch gekennzeichnet, dass** mindestens ein weiterer optischer Bildgeber (23) derart angeordnet ist, dass mindestens ein weiteres der Einzelbilder mit gegenüber der senkrechten Blickrichtung geneigter Blickrichtung aufgenommen wird.

18. Vorrichtung nach Anspruch 17, **dadurch gekennzeichnet, dass** der mindestens eine weitere optische Bildgeber (23) derart geneigt angeordnet ist, dass die Aufnahme in der geneigten Blickrichtung in Kopfrichtung der Fische erfolgt.

19. Vorrichtung nach einem der Ansprüche 11 bis 18, **dadurch gekennzeichnet, dass** die Auswerteeinheit (26) eingerichtet ist, die Einzelbilder der Bildsequenz zu einem Gesamtbild zu überlagern.

20. Vorrichtung zum Entweiden von Fischen (11), die entlang einer Bearbeitungslinie in Längsrichtung (13) mittels einer Fördereinrichtung (12) gefördert werden, umfassend zum Öffnen der Bauchhöhle (10) der Fische eingerichtete Öffnungswerkzeuge, zum Ausräumen der Bauchhöhle (10) ausgebildete Entweidungswerkzeuge und eine Vorrichtung zur anschließenden optischen Inspektion der Bauchhöhle (10) der entweideten Fische (11) nach einem der Ansprüche 11 bis 19.

## Claims

1. Method for the optical inspection of gutted fish (11) with opened abdominal cavity (10), comprising
conveying the fish (11) along a processing line in the longitudinal direction (13) by means of a conveyor device (12),
pivoting of ventral flaps holding elements (17) configured for holding open the abdominal cavity (10) of the fish (11),
taking of at least one sequence of images of abdominal cavity regions of one of the fish (11) in each case, said image sequence comprising a plurality of single images taken using at least one optical imaging system (21, 22, 23) arranged along the processing line,
illuminating the abdominal cavity regions with light of in each case a predetermined spectral composition by means of at least one illumination device (24, 25), in each case at the times the single images are taken,
evaluating the image sequence according to prescribed quality parameters and removal of the relevant fish (11) from the processing line by ejecting it using an ejection device, should the evaluation reveal any deviation from the prescribed quality parameters, wherein
each of the individual images is recorded with a separate optical imaging system (21, 22, 23), wherein
the time interval between the two consecutive single images of one of the image sequences is chosen such that the two consecutive single images at least substantially illustrate the same abdominal cavity region.

2. Method according to claim 1, **characterized in that** the illumination takes place in each case with light of different spectral ranges at the times the images are taken.

3. Method according to claim 2, **characterized in that** the illumination takes place with light of a narrow-band spectrum.

4. Method according to claims 1 to 3, **characterized in that** each of the optical imaging systems (21, 22, 23) is assigned a separate illumination device (24, 25) by means of which the illumination takes place in each case at the times the images are taken.

5. Method according to one of claims 1 to 4, **characterized in that** the single images are taken in black and white.

6. Method according to any one of claims 1 to 5, **characterized in that** at least one of the single images is taken with one of the separate optical imaging systems (21, 22, 23) with a viewing direction onto the abdominal cavity (10) that is vertical or at least substantially vertical.

7. Method according to claim 6, **characterized in that** at least one other of the single images is taken with one other of the optical imaging system (23) with a viewing direction that is inclined in relation to the vertical viewing direction.

8. Method according to claim 7, **characterized in that** in the inclined viewing direction the images are taken towards the head of the fish (11).

9. Method according to any one of claims 2 to 8, **characterized by** overlaying the single images of the image sequence to create an overall image.

10. Method for gutting fish (11) which are conveyed along a processing line in the longitudinal direction (13) by means of a conveying device (12), opening of the abdominal cavity (10) of the fish (11), cleaning out of the abdominal cavity (10) by means of gutting tools and subsequent optical inspection of the abdominal cavity (10) of the gutted fish (11) by means of a method according to any one of claims 1 to 9.

11. Apparatus for optical inspection of the opened abdominal cavity (10) of gutted fish (11), comprising
a conveying device (12) configured for conveying the fish (11) along a processing line in the longitudinal direction,
pivotable ventral flaps holding elements (17) for holding open the abdominal cavity (10) of the fish,
at least one optical imaging system (21, 22, 23) arranged along the processing line which is designed and configured to take at least one sequence of images of abdominal cavity regions of one of the fish (11) in each case, said image sequence comprising a plurality of single images,
at least one illumination device (24, 25) configured for illuminating the abdominal cavity regions, in each case with light of a predetermined spectral composition, in each case at the times the single images are taken,
an evaluation unit (26) configured to evaluate the image sequence according to prescribed quality parameters, said evaluation unit being designed to activate an ejection device to remove the relevant fish (11) from the processing line by ejecting it, should the evaluation reveal any deviation from the prescribed quality parameters, further comprising control means which are designed and configured to control the at least one optical imaging device (21, 22, 23) in such a way that the time interval between taking two consecutive single images of one of the image sequences is chosen such a way that the two consecutive single images at least substantially illustrate the same abdominal cavity region.

12. Apparatus according to claim 11, **characterized in that** the at least one illumination device (24, 25) is configured to illuminate the abdominal cavity region in each case with light of different spectral ranges, at the times the images are taken.

13. Apparatus according to claim 12, **characterized in that** the at least one illumination device (24, 25) comprises a light source which is configured to emit light of a narrow band spectrum.

14. Apparatus according to claim 11, **characterized in that** each of the optical imaging systems (21, 22, 23) is assigned a separate illumination means (24, 25) which is configured to illuminate the abdominal cavity regions in each case at the times the images are taken.

15. Apparatus according to any one of claims 11 to 14, **characterized in that** the at least one optical imaging system (21, 22, 23) is designed as a black-and-white camera.

16. Apparatus according to any one of claims 11 to 15, **characterized in that** the at least one optical imaging system (21, 22, 23) is arranged in such a manner that at least one of the single images is taken with a viewing direction onto the abdominal cavity (10) that is vertical or at least substantially vertical.

17. Apparatus according to claim 16, **characterized in that** at least one further optical imaging system (23) is arranged in such a manner that at least one other of the single images is taken with a viewing direction that is inclined in relation to the vertical viewing direction.

18. Apparatus according to claim 17, **characterized in that** the at least one further optical imaging system (23) is inclined in such a manner that in the inclined viewing direction the images are taken towards the head direction of the fish.

19. Apparatus according to any one of claims 11 to 18, **characterized in that** the evaluation unit (26) is configured to overlay the single images of the image sequence to create an overall image.

20. Apparatus for gutting fish (11) which are conveyed along a processing line in the longitudinal direction (13) by means of a conveying device (12), comprising opening tools configured for opening the abdominal cavity (10) of the fish, gutting tools designed for cleaning out the abdominal cavity (10) and an apparatus for subsequent optical inspection of the abdominal cavity (10) of the gutted fish (11) according to one of claims 11 to 19.

## Revendications

1. Procédé pour l'inspection optique de poissons éviscérés (11) dont la cavité abdominale (10) est ouverte, comprenant
le convoyage des poissons (11) dans la direction longitudinale (13) le long d'une ligne de traitement au moyen d'un appareil de convoyage (12),
le pivotement vers l'intérieur d'éléments de retenue de volet abdominal (17) adaptés pour retenir la cavité abdominale (10) des poissons (11),
l'enregistrement d'au moins une séquence d'images comprenant plusieurs images individuelles de zones de la cavité abdominale de respectivement l'un des poissons (11) au moyen d'au moins un imageur optique (21, 22, 23) agencé le long de la ligne de traitement,
l'éclairage des zones de la cavité abdominale respectivement aux moments d'enregistrement des images individuelles au moyen d'au moins un appareil d'éclairage (24, 25) avec une lumière de composition spectrale respectivement prédéterminée,
l'évaluation de la séquence d'images en ce qui concerne des paramètres de qualité prédéfinis et, si l'évaluation révèle un écart par rapport aux paramètres de qualité prédéfinis, l'enlèvement de chacun des poissons (11) de la ligne de traitement par éjection au moyen d'un appareil d'éjection,
chacune des images individuelles étant enregistrée avec un imageur optique séparé (21, 22, 23),
l'intervalle de temps entre l'enregistrement de deux images individuelles successives d'une des séquences d'images étant choisi de telle sorte que les deux images individuelles successives représentent au moins essentiellement la même zone de la cavité abdominale.

2. Procédé selon la revendication 1, **caractérisé en ce que** l'éclairage aux moments d'enregistrement s'effectue respectivement avec de la lumière de différentes plages spectrales.

3. Procédé selon la revendication 2, **caractérisé en ce que** l'éclairage s'effectue avec de la lumière d'un spectre à bande étroite.

4. Procédé selon les revendications 1 à 3, **caractérisé en ce qu'**un appareil d'éclairage séparé (24, 25) est associé à chacun des imageurs optiques (21, 22, 23), au moyen duquel l'éclairage est effectué aux moments d'enregistrement respectifs.

5. Procédé selon l'une quelconque des revendications 1 à 4, **caractérisé en ce que** l'enregistrement des images individuelles est effectué en noir et blanc.

6. Procédé selon l'une quelconque des revendications 1 à 5, **caractérisé en ce qu'**au moins l'une des images individuelles est enregistrée avec l'un des imageurs optiques (21, 22, 23) avec une direction de vision verticale ou au moins essentiellement verticale sur la cavité abdominale (10).

7. Procédé selon la revendication 6, **caractérisé en ce qu'**au moins une autre des images individuelles est enregistrée avec un autre des imageurs optiques (23) avec une direction de vision inclinée par rapport à la direction de vision verticale.

8. Procédé selon la revendication 7, **caractérisé en ce que** l'enregistrement dans la direction de vision inclinée est effectué dans la direction de la tête des poissons (11).

9. Procédé selon l'une quelconque des revendications 2 à 8, **caractérisé par** la superposition des images individuelles de la séquence d'images en une image globale.

10. Procédé pour l'éviscération de poissons (11), qui sont transportés le long d'une ligne de traitement dans la direction longitudinale (13) au moyen d'un appareil de convoyage (12), l'ouverture de la cavité abdominale (10) des poissons (11), le vidage de la cavité abdominale (10) au moyen d'outils d'éviscération, puis l'inspection optique de la cavité abdominale (10) des poissons éviscérés (11) au moyen d'un procédé selon l'une quelconque des revendications 1 à 9.

11. Dispositif pour l'inspection optique de la cavité abdominale ouverte (10) de poissons éviscérés (11), comprenant
un appareil de convoyage (12) adapté pour transporter les poissons (11) dans la direction longitudinale le long d'une ligne de traitement,
des éléments de retenue de volet abdominal (17) pivotants adaptés pour retenir la cavité abdominale (10) des poissons,
au moins un imageur optique (21, 22, 23) agencé le long de la ligne de traitement, qui est configuré et adapté pour enregistrer au moins une séquence d'images comprenant plusieurs images individuelles de zones de la cavité abdominale de respectivement l'un des poissons (11),
au moins un appareil d'éclairage (24, 25) adapté pour éclairer les zones de la cavité abdominale respectivement aux moments d'enregistrement des images individuelles avec une lumière de composition spectrale respectivement prédéterminée,
une unité d'évaluation (26) adaptée pour évaluer la séquence d'images en ce qui concerne des paramètres de qualité prédéfinis, qui est configurée, si l'évaluation révèle un écart par rapport aux paramètres de qualité prédéfinis, pour commander un appareil d'éjection afin d'enlever le poisson respectif (11) de la ligne de traitement par éjection,
plusieurs des imageurs optiques (21, 22, 23) étant agencés séparément le long de la ligne de traitement et étant adaptés pour enregistrer chacune des images individuelles avec l'un des imageurs optiques (21, 22, 23), ainsi que
des moyens de commande qui sont configurés et adaptés pour commander l'au moins un imageur optique (21, 22, 23) de telle sorte que l'intervalle de temps entre les enregistrements de deux images individuelles successives d'une des séquences d'images soit choisi de telle sorte que les deux images individuelles successives représentent au moins essentiellement la même zone de la cavité abdominale.

12. Dispositif selon la revendication 11, **caractérisé en ce que** l'au moins un appareil d'éclairage (24, 25) est adapté pour éclairer la zone de la cavité abdominale aux moments d'enregistrement respectivement avec de la lumière de différentes plages spectrales.

13. Dispositif selon la revendication 12, **caractérisé en ce que** l'au moins un appareil d'éclairage (24, 25) comprend une source lumineuse qui est configurée pour émettre de la lumière d'un spectre à bande étroite.

14. Dispositif selon la revendication 11, **caractérisé en ce qu'**un moyen d'éclairage (24, 25) séparé est associé à chacun des imageurs optiques (21, 22, 23), qui est adapté pour éclairer les zones de la cavité abdominale aux moments d'enregistrement respectifs.

15. Dispositif selon l'une quelconque des revendications 11 à 14, **caractérisé en ce que** l'au moins un imageur optique (21, 22, 23) est configuré sous forme de caméra noir et blanc.

16. Dispositif selon l'une quelconque des revendications 11 à 15, **caractérisé en ce que** l'au moins un imageur optique (21, 22, 23) est agencé de telle sorte qu'au moins l'une des images individuelles est enregistrée avec une direction de vision verticale ou au moins essentiellement verticale sur la cavité abdominale (10).

17. Dispositif selon la revendication 16, **caractérisé en ce qu'**au moins un autre imageur optique (23) est agencé de telle sorte qu'au moins une autre des images individuelles est enregistrée avec une direction de vision inclinée par rapport à la direction de vision verticale.

18. Dispositif selon la revendication 17, **caractérisé en ce que** l'au moins un autre imageur optique (23) est agencé de manière inclinée de telle sorte que l'enregistrement dans la direction de vision inclinée s'effectue dans la direction de la tête des poissons.

19. Dispositif selon l'une quelconque des revendications 11 à 18, **caractérisé en ce que** l'unité d'évaluation (26) est adaptée pour superposer les images individuelles de la séquence d'images en une image globale.

20. Dispositif pour l'éviscération de poissons (11), qui sont transportés le long d'une ligne de traitement dans la direction longitudinale (13) au moyen d'un appareil de convoyage (12), comprenant des outils d'ouverture adaptés pour ouvrir la cavité abdominale (10) des poissons, des outils d'éviscération configurés pour vider la cavité abdominale (10) et un dispositif pour l'inspection optique ultérieure de la cavité abdominale (10) des poissons éviscérés (11) selon l'une quelconque des revendications 11 à 19.
